# EUROPÄISCHE PATENTANMELDUNG

(11) **EP 4 529 848 A2**
(43) Veröffentlichungstag der Anmeldung: **02.04.2025**
(21) Anmeldenummer: 25157346.5
(22) Anmeldetag: 31.03.2023
(51) Int. Cl.: A61B 6/00

(54) **SYSTEM MIT COMPUTERTOMOGRAPHIE-GANTRY UND HUBVORRICHTUNG**

(62) Teilanmeldung aus: 23166130.7
(71) Anmelder: Siemens Healthineers AG, 91301 Forchheim (DE)
(72) Erfinder: Atzinger, Michael, 95517 Seybothenreuth (DE); Groß, Stefan, 92724 Trabitz (DE); Gärtner, Ralf, 91126 Schwabach (DE); Hesl, Stefan, 92676 Eschenbach (DE); Hruschka, Klaus, 92681 Erbendorf (DE); Hupfauf, Matthias, 92507 Nabburg (DE); Kassler, Ulli Alfred, 90419 Nürnberg (DE); Kiesel, Jan-Christoph, 24354 Kosel (DE); Krug, Rita, 90762 Fürth (DE); Neuber, Wolfgang, 92676 Eschenbach i. d. Opf. (DE); Raschke, Daniel, 95447 Bayreuth (DE); Walberer, Georg, 95506 Kastl (DE)
(74) Vertreter: Siemens Healthineers Patent Attorneys

(57) **Zusammenfassung**

Die Erfindung betrifft ein System, aufweisend einen Fahrwagen, in den eine Computertomographie-Gantry aufnehmbar ist, ein Schienensystem, ein Drehlager und eine Hubvorrichtung,
- wobei in einem Translations-Betriebszustand des Systems der Fahrwagen entlang des Schienensystems derart bewegbar, insbesondere rollend bewegbar, gelagert ist, dass entlang des Schienensystems eine erste Translationsbewegung des Fahrwagens ausführbar ist, wobei das Drehlager und die Hubvorrichtung jeweils derart in den Fahrwagen aufgenommen sind, dass sie der ersten Translationsbewegung des Fahrwagens folgen,
- wobei in einem Übergangs-Betriebszustand des Systems der Fahrwagen mittels der Hubvorrichtung relativ zu dem Schienensystem entlang einer vertikalen Drehachse derart bewegbar gelagert ist, dass entlang der vertikalen Drehachse eine Hubbewegung des Fahrwagens relativ zu dem Schienensystem ausführbar ist,
- wobei die Computertomographie-Gantry der ersten Translationsbewegung des Fahrwagens und der Hubbewegung des Fahrwagens relativ zu dem Schienensystem folgt, wenn die Computertomographie-Gantry in den Fahrwagen aufgenommen ist.

## Beschreibung

Die Erfindung betrifft ein System und ein Verfahren zum Bewegen eines Fahrwagens.

Ein Computertomographie-System (CT-System) wird manchmal für zwei benachbarte Behandlungsräume verwendet. Grund hierfür ist einerseits der Kostenaspekt, um nicht für jeden der beiden Räume ein CT-System installieren zu müssen. Andererseits bietet ein verfahrbares und in einen anderen Raum bewegbares CT-System auch in Bezug auf den Platzbedarf Vorteile. Als ein Beispiel für ein verfahrbares CT-System sei auf die WO 2011/112307 A1 verwiesen.

Bei der Bediendung von zwei gegenüberliegenden Behandlungsräumen können die Patientenliegen beispielsweise kopfseitig zueinander angeordnet. Das CT-System kann somit zwischen den beiden Patientenliegen hin- und herfahren, sodass die Computertomographie-Gantry in dem einen Behandlungsraum mit einer Seite zu der Patientenliege weisend angeordnet wird und in dem gegenüberliegenden Behandlungsraum mit der anderen Seite zu der Patientenliege weisend angeordnet wird. Diese unterschiedlichen Anordnungen der Computertomographie-Gantry relativ zur jeweiligen Patientenliege sind bei der Verarbeitung der Bildgebungsdaten zu berücksichtigen.

Bei manchen CT-Systemen ist die Scanebene im Wesentlichen mittig bzw. zentrisch im Gehäuse der Computertomographie-Gantry angeordnet. Bei einigen CT-Systemen ist die Scanebene jedoch nicht mittig im Gehäuse der Computertomographie-Gantry angeordnet. Es gibt also eine Vorderseite der Computertomographie-Gantry und eine Rückseite der Computertomographie-Gantry. Für die Bedienung von zwei gegenüberliegenden Behandlungsräumen wäre es dann vorteilhaft, die Computertomographie-Gantry stets mit der Vorderseite zur jeweiligen Patientenliege weisend auszurichten. Die Computertomographie-Gantry muss also für eine Zweiraumlösung mit zwei zueinander kopfseitig angeordneten Patientenliegen um 180° gedreht werden können, um jeweils mit ihrer Vorderseite zur jeweiligen Patientenliege weisend ausgerichtet zu sein.

Die Erfindung hat die Aufgabe, eine Alternative zu einem herkömmlichen Bewegen eines Fahrwagens, in den eine Computertomographie-Gantry aufnehmbar ist, bereitzustellen.

Jeder Gegenstand eines unabhängigen Anspruchs löst diese Aufgabe. In den abhängigen Ansprüchen sind weitere vorteilhafte Aspekte der Erfindung berücksichtigt. Unabhängig vom grammatikalischen Geschlecht eines bestimmten Begriffes sind Personen mit männlicher, weiblicher oder anderer Geschlechteridentität mit umfasst.

Diese Offenbarung betrifft ferner ein System, aufweisend eine Computertomographie-Gantry, einen Fahrwagen, ein Schienensystem, ein Drehlager und eine Hubvorrichtung,
- wobei in einem Translations-Betriebszustand des Systems der Fahrwagen entlang des Schienensystems derart bewegbar, insbesondere rollend bewegbar, gelagert ist, dass entlang des Schienensystems eine erste Translationsbewegung des Fahrwagens ausführbar ist, wobei die Computertomographie-Gantry, das Drehlager und die Hubvorrichtung jeweils derart in den Fahrwagen aufgenommen sind, dass sie der ersten Translationsbewegung des Fahrwagens folgen,
- wobei in einem Übergangs-Betriebszustand des Systems der Fahrwagen mittels der Hubvorrichtung relativ zu dem Schienensystem entlang einer vertikalen Drehachse derart bewegbar gelagert ist, dass entlang der vertikalen Drehachse eine Hubbewegung des Fahrwagens relativ zu dem Schienensystem ausführbar ist, wobei die Computertomographie-Gantry derart in den Fahrwagen aufgenommen ist, dass sie der Hubbewegung des Fahrwagens relativ zu dem Schienensystem folgt,
- wobei in einem Rotations-Betriebszustand des Systems der Fahrwagen mittels der Hubvorrichtung relativ zu dem Schienensystem entlang der vertikalen Drehachse derart angehoben ist, dass der Fahrwagen von dem Schienensystem losgelöst ist, und der Fahrwagen mittels des Drehlagers relativ zu dem Schienensystem um die vertikale Drehachse derart drehbar gelagert ist, dass eine Rotationsbewegung des Fahrwagens relativ zu dem Schienensystem um die vertikale Drehachse ausführbar ist, wobei die Computertomographie-Gantry derart in den Fahrwagen aufgenommen ist, dass sie der Rotationsbewegung des Fahrwagens relativ zu dem Schienensystem um die vertikale Drehachse folgt.

Insbesondere kann vorgesehen sein, dass in dem Übergangs-Betriebszustand des Systems der Fahrwagen mittels der Hubvorrichtung relativ zu dem Schienensystem entlang der vertikalen Drehachse derart bewegbar gelagert ist, dass entlang der vertikalen Drehachse eine Senkbewegung des Fahrwagens relativ zu dem Schienensystem ausführbar ist, wobei die Computertomographie-Gantry derart in den Fahrwagen aufgenommen ist, dass sie der Senkbewegung des Fahrwagens relativ zu dem Schienensystem folgt.

Insbesondere kann vorgesehen sein, dass das System einen Fahrantrieb und/oder einen Drehantrieb aufweist. Der Fahrantrieb kann insbesondere zum Antreiben der ersten Translationsbewegung des Fahrwagens und/oder zum Antreiben der zweiten Translationsbewegung des Fahrwagens eingerichtet sein. Der Drehantrieb kann insbesondere zum Antreiben der Rotationsbewegung des Fahrwagens relativ zu dem Schienensystem um die vertikale Drehachse eingerichtet sein. Das Drehlager kann beispielsweise ein Wälzlager, insbesondere ein Axial-Wälzlager, sein. Statt einen Drehantrieb vorzusehen, kann das System auch für ein manuelles Antreiben der Rotationsbewegung des Fahrwagens relativ zu dem Schienensystem um die vertikale Drehachse, beispielsweise durch die Körperkraft einer Bedienperson eingerichtet sein.

Die Hubvorrichtung kann beispielsweise einen Satz von Hubzylindern und/oder einen Hub-Antrieb aufweisen. Insbesondere kann vorgesehen sein, dass das Drehlager in Bezug auf eine horizontale Richtung zwischen zwei Hubzylindern des Satzes von Hubzylindern angeordnet ist und/oder dass die Hubzylinder des Satzes von Hubzylindern beispielsweise mittels einer Welle miteinander synchronisiert sind. Die horizontale Richtung kann insbesondere zu der ersten Translationsbewegung im Wesentlichen senkrecht sein.

Beispielsweise kann damit die Computertomographie-Gantry derart drehbar, insbesondere derart um 180° drehbar, angeordnet sein, dass in zwei gegenüberliegenden Behandlungsräumen jede von zwei zueinander kopfseitig angeordneten Patientenliegen jeweils mit der Vorderseite der Computertomographie-Gantry angefahren werden kann. Die beiden Behandlungsräume würden dann in Bezug auf die Erzeugung der Bildgebungsdaten, soweit es die Orientierung der Computertomographie-Gantry relativ zu der jeweiligen Patientenliege betrifft, gleichwertig sein.

Insbesondere kann vorgesehen sein, dass das System eine Grundfläche aufweist. Das Schienensystem kann insbesondere relativ zu der Grundfläche stationär und/oder in der Grundfläche fest verankert sein. Die Grundfläche kann beispielsweise ein Boden, insbesondere ein Boden eines Behandlungsraumes, sein und/oder eine Bodenplatte und/oder eine Unterlage aufweisen. Insbesondere kann vorgesehen sein, dass die Oberfläche der Grundfläche im Wesentlichen horizontal ist, insbesondere horizontal ist.

Die erste Translationsbewegung kann insbesondere horizontal und/oder zu der Grundfläche parallel sein. Die zweite Translationsbewegung kann insbesondere horizontal und/oder zu der Grundfläche parallel sein. Die erste Translationsbewegung kann beispielsweise auf einem geraden ersten Pfad und/oder auf einem gekrümmten ersten Pfad erfolgen. Die zweite Translationsbewegung kann beispielsweise auf einem geraden zweiten Pfad und/oder auf einem gekrümmten zweiten Pfad erfolgen. Das Schienensystem kann insbesondere für eine Translation des Fahrwagens zwischen zwei Behandlungsräumen eingerichtet sein.

Das System kann insbesondere eine Kabelführung aufweisen, die dazu eingerichtet ist, die Computertomographie-Gantry und/oder den Fahrwagen an eine relativ zu der Grundfläche stationäre Energieübertragungseinrichtung und/oder an eine relativ zu der Grundfläche stationäre Datenübertragungseinrichtung anzubinden, insbesondere während der ersten horizontalen Translationsbewegung des Fahrwagens und/oder während der Rotationsbewegung des Fahrwagens relativ zu dem Schienensystem anzubinden. Die Kabelführung kann beispielsweise deckenbasiert und/oder bodenbasiert sein. Die Kabelführung kann beispielsweise eine fest mit dem Fahrwagen und/oder mit der Computertomographie-Gantry verbundene Kabelsäule aufweisen, welche flexibel mit der relativ zu der Grundfläche stationären Energieübertragungseinrichtung und/oder mit der relativ zu der Grundfläche stationären Datenübertragungseinrichtung verbunden ist. Das System kann beispielsweise eine Übertragungsschnittstelle zur Übertragung, insbesondere zur bidirektionalen Übertragung, von Energie und/oder Daten zwischen dem Fahrwagen und der Computertomographie-Gantry aufweisen. Die Übertragungsschnittstelle kann beispielsweise kontaktbasiert und/oder kontaktlos sein.

Insbesondere kann vorgesehen sein, dass ein Untersuchungsobjekt relativ zu dem Schienensystem und/oder relativ zu der Grundfläche ruht. Das Schienensystem kann insbesondere eine Linearführung für den Fahrwagen bilden.

Beispielsweise kann das System einen Untersuchungstisch zur Lagerung eines Untersuchungsobjekts aufweisen. Der Untersuchungstisch kann insbesondere relativ zu dem Schienensystem und/oder relativ zu der Grundfläche ruhen und/oder relativ zu dem Schienensystem und/oder relativ zu der Grundfläche fest verankert sein. Das Untersuchungsobjekt kann beispielsweise eine zu untersuchende Person, insbesondere ein Patient, sein und/oder auf dem Untersuchungstisch gelagert sein, insbesondere relativ zu dem Untersuchungstisch ruhend gelagert sein.

Die Computertomographie-Gantry kann beispielsweise einen Tragrahmen und einen relativ zu dem Tragrahmen drehbar gelagerten Rotor aufweisen, wobei die Strahlungsquelle und der Strahlungsdetektor an dem Rotor angeordnet sind. Optional kann die Computertomographie-Gantry einen relativ zu dem Tragrahmen kippbar gelagerten Kipprahmen aufweisen, wobei der Rotor an dem Kipprahmen angeordnet ist. Die Strahlungsquelle und der Strahlungsdetektor können für eine Aufnahme eines Projektionsdatensatzes von dem Untersuchungsobjekt zusammenwirken. Die Computertomographie-Gantry kann beispielsweise eine Öffnung aufweisen. Insbesondere können das Schienensystem, der Untersuchungstisch und die Öffnung derart zueinander angeordnet sein, dass durch die erste Translationsbewegung der Computertomographie-Gantry der Untersuchungstisch in die Öffnung eingeführt wird, insbesondere zusammen mit dem auf dem Untersuchungstisch gelagerten Untersuchungsobjekt in die Öffnung eingeführt wird.

Eine Ausführungsform sieht vor, dass das Schienensystem einen Satz von Schienen aufweist, wobei der Fahrwagen einen Satz von Rädern aufweist, wobei die Rotationsbewegung des Fahrwagens relativ zu dem Schienensystem um die vertikale Drehachse von einem ersten Winkel um die vertikale Drehachse bis zu einem zweiten Winkel um die vertikale Drehachse erfolgt, wobei der Satz von Rädern in Bezug auf die vertikale Drehachse derart angeordnet ist, dass der Satz von Rädern auf dem Satz von Schienen rollen kann, wenn der Fahrwagen relativ zu dem Schienensystem in dem ersten Winkel um die vertikale Drehachse angeordnet ist, und dass der Satz von Rädern auf dem Satz von Schienen rollen kann, wenn der Fahrwagen relativ zu dem Schienensystem in dem zweiten Winkel um die vertikale Drehachse angeordnet ist.

Beispielsweise kann der Fahrwagen für jedes Rad des Satzes von Rädern einen Rad-Direktantrieb, der mit diesem Rad zusammenwirkt, aufweisen. Der Rad-Direktantrieb kann beispielsweise einen Elektromotor, insbesondere einen Elektro-Radnabenmotor, aufweisen. Insbesondere kann vorgesehen sein, dass die Rad-Direktantriebe der Räder des Satzes von Rädern zusammen den Fahrantrieb bilden.

Insbesondere kann vorgesehen sein, dass der Satz von Schienen und der Satz von Rädern einen Satz von Rad-Schiene-Rollkontakten bilden. Insbesondere kann vorgesehen sein, dass jede Schiene des Satzes von Schienen eine Rundschiene ist und/oder dass jedes Rad des Satzes von Rädern eine konkave Rolle und/oder zum Abrollen auf einer Rundschiene ausgebildet ist. Die Schienen und/oder die Räder können beispielsweise aus Stahl hergestellt sein. Die Rundschienen können insbesondere ohne Abdeckung und ohne Antriebselemente im Boden integriert werden und dabei ein Überfahren mit Patientenbetten und Instrumententischen ermöglichen.

Eine Ausführungsform sieht vor, dass ein Absolutbetrag einer Winkeldifferenz zwischen dem ersten Winkel um die vertikale Drehachse und dem zweiten Winkel um die vertikale Drehachse größer als 0° und kleiner als 360° ist, insbesondere größer als 10° und kleiner als 350° ist, beispielsweise größer als 175° und kleiner als 185° ist, insbesondere gleich 180° Grad ist.

Eine Ausführungsform sieht vor, dass das System ferner eine Stützstruktur aufweist, wobei das Schienensystem relativ zu der Stützstruktur ruht, wobei in dem Translations-Betriebszustand des Systems der Fahrwagen entlang des Schienensystems derart relativ zu der Stützstruktur bewegbar gelagert ist, dass entlang des Schienensystems die erste Translationsbewegung des Fahrwagens relativ zu der Stützstruktur ausführbar ist, wobei in dem Übergangs-Betriebszustand des Systems und in dem Rotations-Betriebszustand des Systems der Fahrwagen mittels der Hubvorrichtung und des Drehlagers auf die Stützstruktur abgestützt ist.

Insbesondere kann vorgesehen sein, dass das Schienensystem relativ zu der Stützstruktur ruht, während sich das System in dem Translations-Betriebszustand des Systems befindet, während sich das System in dem Übergangs-Betriebszustand des Systems befindet und während sich das System in dem Rotations-Betriebszustand des Systems befindet.

Die Stützstruktur kann insbesondere in Bezug auf die vertikale Drehachse unterhalb des Fahrwagens angeordnet sein. Die Stützstruktur kann beispielsweise ein Bereich der Grundfläche und/oder eine Stützplatte sein. Beispielsweise kann vorgesehen sein, dass die Stützplatte relativ zu der Grundfläche fest verankert ist und/oder in die Grundfläche eingelassen ist, insbesondere derart eingelassen ist, dass eine Oberfläche der Stützplatte an eine Oberfläche der Grundfläche plan anschließt.

Insbesondere kann vorgesehen sein, dass der Fahrwagen durch die erste Translationsbewegung in eine Drehposition relativ zu der Stützstruktur gebracht werden kann, in welcher durch ein Abstützen der Hubvorrichtung auf die Stützstruktur ein Anheben des Fahrwagens mittels der Hubvorrichtung relativ zu der Stützstruktur entlang der vertikalen Drehachse erfolgen kann.

Eine Ausführungsform sieht vor, dass das System eine Sockelstruktur aufweist, wobei die Sockelstruktur mittels der Hubvorrichtung relativ zu dem Fahrwagen entlang der vertikalen Drehachse bewegbar gelagert ist, wobei die Sockelstruktur mittels des Drehlagers relativ zu dem Fahrwagen um die vertikale Drehachse drehbar gelagert ist. Insbesondere kann vorgesehen sein, dass die Sockelstruktur mittels des Drehlagers relativ mit der Hubvorrichtung verbunden ist und relativ zu der Hubvorrichtung um die vertikale Drehachse drehbar gelagert ist.

Die Sockelstruktur kann insbesondere derart mittels der Hubvorrichtung und des Drehlagers mit dem Fahrwagen verbunden sein, dass die Sockelstruktur der ersten Translationsbewegung des Fahrwagens folgt. Die Hubvorrichtung kann in den Fahrwagen insbesondere derart aufgenommen sein, dass eine Erstreckung der Hubvorrichtung entlang der vertikalen Drehachse veränderbar ist. Insbesondere kann vorgesehen sein, dass die Erstreckung der Hubvorrichtung entlang der vertikalen Drehachse in dem Rotations-Betriebszustand des Systems größer ist als in dem Translations-Betriebszustand des Systems ist.

Das System kann insbesondere eine Verriegelungseinheit aufweisen, wobei in dem Translations-Betriebszustand des Systems die Sockelstruktur mittels der Verriegelungseinheit gegen eine Winkeländerung um die vertikale Drehachse relativ zu dem Fahrwagen gesichert ist.

Insbesondere kann vorgesehen sein, dass die Hubvorrichtung dazu eingerichtet ist, ausgehend von dem Translations-Betriebszustand des Systems einen Abstand zwischen der Sockelstruktur und dem Fahrwagen entlang der vertikalen Drehachse zu vergrößern, insbesondere derart zu vergrößern, dass dadurch eine Lücke zwischen der Sockelstruktur und der Stützstruktur verkleinert wird, und durch ein Stemmen der Sockelstruktur gegen die Stützstruktur den Fahrwagen relativ zu dem Schienensystem entlang der vertikalen Drehachse derart anzuheben, dass der Fahrwagen von dem Schienensystem losgelöst ist, wobei durch das Stemmen der Sockelstruktur gegen die Stützstruktur die Sockelstruktur derart gegen eine Winkeländerung um die vertikale Drehachse relativ zu der Stützstruktur fixiert ist, dass der Fahrwagen mittels des Drehlagers relativ zu der Stützstruktur und damit insbesondere relativ zu dem Schienensystem um die vertikale Drehachse drehbar gelagert ist, sodass das System in den Rotations-Betriebszustand des Systems übergeht.

Eine Ausführungsform sieht vor, dass durch das Stemmen der Sockelstruktur gegen die Stützstruktur die Sockelstruktur derart gegen die Winkeländerung um die vertikale Drehachse relativ zu der Stützstruktur reibschlüssig fixiert ist, dass der Fahrwagen mittels des Drehlagers relativ zu der Stützstruktur und damit insbesondere relativ zu dem Schienensystem um die vertikale Drehachse drehbar gelagert ist.

Eine Ausführungsform sieht vor, dass eine Oberseite der Stützstruktur im Wesentlichen plan ist, insbesondere horizontal ist, und/oder dass eine Unterseite der Sockelstruktur im Wesentlichen plan ist, insbesondere horizontal ist. Insbesondere kann vorgesehen sein, dass das Stemmen der Sockelstruktur gegen die Stützstruktur einen Reibschluss zwischen der Oberseite der Stützstruktur und der Unterseite der Sockelstruktur bewirkt, wobei durch den Reibschluss die Sockelstruktur derart gegen die Winkeländerung um die vertikale Drehachse relativ zu der Stützstruktur reibschlüssig fixiert ist, dass der Fahrwagen mittels des Drehlagers relativ zu der Stützstruktur und damit insbesondere relativ zu dem Schienensystem um die vertikale Drehachse drehbar gelagert ist.

Eine Ausführungsform sieht vor, dass die Hubvorrichtung dazu eingerichtet ist, ausgehend von dem Rotations-Betriebszustand des Systems den Abstand zwischen der Sockelstruktur und dem Fahrwagen entlang der vertikalen Drehachse zu verkleinern, insbesondere derart zu verkleinern, dass dadurch die Lücke zwischen der Sockelstruktur und der Stützstruktur vergrößert wird, und dadurch den Fahrwagen relativ zu dem Schienensystem entlang der vertikalen Drehachse bis zu einem Abstellen des Fahrwagens auf dem Schienensystem abzusenken, sodass das System in den Translations-Betriebszustand des Systems übergeht.

Eine Ausführungsform sieht vor, dass die Hubvorrichtung und das Drehlager direkt aneinandergekoppelt sind, sodass sie ein Hub-Dreh-Modul bilden, wobei der Fahrwagen eine Aussparung zur Aufnahme des Hub-Dreh-Moduls aufweist, wobei das Hub-Dreh-Modul in der Aussparung derart angeordnet ist, dass sich die Hubvorrichtung entlang der vertikalen Drehachse nach unten aus der Aussparung herausstrecken kann, um den Fahrwagen relativ zu dem Schienensystem entlang der vertikalen Drehachse anzuheben. Das Hub-Dreh-Modul ist über eine mechanische Schnittstelle und eine elektrische Schnittstelle mit dem Fahrwagen lösbar verbunden. Dadurch kann das Hub-Dreh-Modul mit geringem Aufwand von dem Fahrwagen getrennt bzw. darin montiert werden. Das Hub-Dreh-Modul kann insbesondere in Form eines Stempels ausgebildet sein.

Diese Offenbarung betrifft ferner ein Verfahren zum Bewegen einer Computertomographie-Gantry, das Verfahren umfassend:
- ein Ausführen einer ersten Translationsbewegung eines Fahrwagens entlang eines Schienensystems, während sich ein System, welches die Computertomographie-Gantry, den Fahrwagen, das Schienensystem, ein Drehlager und eine Hubvorrichtung aufweist, in einem Translations-Betriebszustand des Systems befindet, wobei in dem Translations-Betriebszustand des Systems der Fahrwagen entlang des Schienensystems bewegbar gelagert ist, wobei die Computertomographie-Gantry, das Drehlager und die Hubvorrichtung jeweils derart in den Fahrwagen aufgenommen sind, dass sie der ersten Translationsbewegung des Fahrwagens folgen,
- ein Ausführen einer Hubbewegung des Fahrwagens relativ zu dem Schienensystem entlang einer vertikalen Drehachse, während sich das System in einem Übergangs-Betriebszustand des Systems befindet, wobei in dem Übergangs-Betriebszustand des Systems der Fahrwagen mittels der Hubvorrichtung relativ zu dem Schienensystem entlang einer vertikalen Drehachse bewegbar gelagert ist, wobei die Computertomographie-Gantry derart in den Fahrwagen aufgenommen ist, dass sie der Hubbewegung des Fahrwagens relativ zu dem Schienensystem folgt,
- ein Ausführen einer Rotationsbewegung des Fahrwagens relativ zu dem Schienensystem um die vertikale Drehachse, während sich das System in einem Rotations-Betriebszustand des Systems befindet, wobei in dem Rotations-Betriebszustand des Systems der Fahrwagen mittels der Hubvorrichtung relativ zu dem Schienensystem entlang der vertikalen Drehachse derart angehoben ist, dass der Fahrwagen von dem Schienensystem losgelöst ist, und der Fahrwagen mittels des Drehlagers relativ zu dem Schienensystem um die vertikale Drehachse drehbar gelagert ist, wobei die Computertomographie-Gantry derart in den Fahrwagen aufgenommen ist, dass sie der Rotationsbewegung des Fahrwagens relativ zu dem Schienensystem um die vertikale Drehachse folgt.

Eine Ausführungsform sieht vor, dass nach dem Ausführen der Rotationsbewegung des Fahrwagens relativ zu dem Schienensystem um die vertikale Drehachse eine Senkbewegung des Fahrwagens relativ zu dem Schienensystem entlang der vertikalen Drehachse ausgeführt wird, während sich das System in dem Übergangs-Betriebszustand des Systems befindet, wobei die Computertomographie-Gantry derart in den Fahrwagen aufgenommen ist, dass sie der Senkbewegung des Fahrwagens relativ zu dem Schienensystem folgt.

Eine Ausführungsform sieht vor, dass nach einem Ausführen der Senkbewegung des Fahrwagens relativ zu dem Schienensystem entlang der vertikalen Drehachse eine zweite Translationsbewegung des Fahrwagens entlang des Schienensystems ausgeführt wird, während sich das System in dem Translations-Betriebszustand des Systems befindet, wobei die Computertomographie-Gantry, das Drehlager und die Hubvorrichtung jeweils derart in den Fahrwagen aufgenommen sind, dass sie der zweiten Translationsbewegung des Fahrwagens folgen.

Insbesondere kann vorgesehen sein, dass das Schienensystem einen Satz von Schienen aufweist, wobei der Fahrwagen einen Satz von Rädern aufweist, wobei die Rotationsbewegung des Fahrwagens relativ zu dem Schienensystem um die vertikale Drehachse von einem ersten Winkel um die vertikale Drehachse bis zu einem zweiten Winkel um die vertikale Drehachse erfolgt, wobei während der ersten Translationsbewegung des Fahrwagens entlang des Schienensystems der Fahrwagen relativ zu dem Schienensystem in dem ersten Winkel um die vertikale Drehachse angeordnet ist und der Satz von Rädern auf dem Satz von Schienen rollt, wobei während der zweiten Translationsbewegung des Fahrwagens entlang des Schienensystems der Fahrwagen relativ zu dem Schienensystem in dem zweiten Winkel um die vertikale Drehachse angeordnet ist und der Satz von Rädern auf dem Satz von Schienen rollt.

Weiterhin kann vorgesehen sein, dass nach der Rotationsbewegung des Fahrwagens relativ zu dem Schienensystem um die vertikale Drehachse der Satz von Rädern durch ein Absenken des Fahrwagens entlang der vertikalen Drehachse auf dem Satz von Schienen rollend angeordnet werden kann.

Eine Ausführungsform sieht vor, dass das System ferner eine Stützstruktur aufweist, wobei das Schienensystem relativ zu der Stützstruktur ruht, wobei die erste Translationsbewegung des Fahrwagens entlang des Schienensystems relativ zu der Stützstruktur ausgeführt wird, während sich das System in dem Translations-Betriebszustand des Systems befindet, wobei nach dem Ausführen der ersten Translationsbewegung des Fahrwagens entlang des Schienensystems relativ zu der Stützstruktur der Fahrwagen mittels der Hubvorrichtung und des Drehlagers auf die Stützstruktur abgestützt wird, insbesondere derart abgestützt wird, dass das System in den Übergangs-Betriebszustand des Systems übergeht.

Eine Ausführungsform sieht vor, dass das System eine Sockelstruktur aufweist, wobei die Sockelstruktur mittels der Hubvorrichtung relativ zu dem Fahrwagen entlang der vertikalen Drehachse bewegbar gelagert ist, wobei die Sockelstruktur mittels des Drehlagers relativ zu dem Fahrwagen um die vertikale Drehachse drehbar gelagert ist.

Insbesondere kann vorgesehen sein, dass ausgehend von dem Translations-Betriebszustand des Systems mittels der Hubvorrichtung ein Abstand zwischen der Sockelstruktur und dem Fahrwagen entlang der vertikalen Drehachse vergrößert wird und durch ein Stemmen der Sockelstruktur gegen die Stützstruktur der Fahrwagen relativ zu dem Schienensystem entlang der vertikalen Drehachse derart angehoben wird, dass der Fahrwagen von dem Schienensystem losgelöst wird, wobei durch das Stemmen der Sockelstruktur gegen die Stützstruktur die Sockelstruktur gegen eine Drehung um die vertikale Drehachse relativ zu der Stützstruktur fixiert wird, insbesondere derart fixiert wird dass der Fahrwagen mittels des Drehlagers relativ zu der Stützstruktur und damit insbesondere relativ zu dem Schienensystem um die vertikale Drehachse drehbar gelagert ist, sodass das System in den Rotations-Betriebszustand des Systems übergeht.

Eine Ausführungsform sieht vor, dass durch das Stemmen der Sockelstruktur gegen die Stützstruktur die Sockelstruktur gegen eine Drehung um die vertikale Drehachse relativ zu der Stützstruktur reibschlüssig fixiert wird.

Weiterhin kann vorgesehen sein, dass das System für Beschleunigungen der Computertomographie-Gantry in Scan-Richtung von 265 mm/s² beim Scan-Vorgang eingerichtet ist, dass das System für Beschleunigungen der Computertomographie-Gantry in Scan-Richtung von 1400 mm/s² beim Nothalt eingerichtet ist, und/oder dass die Rotationsbewegung des Fahrwagens relativ zu dem Schienensystem um die vertikale Drehachse in weniger als 60 Sekunden, insbesondere in weniger als 40 Sekunden, beispielsweise in ungefähr 30 Sekunden, abgeschlossen werden kann.

Die Verkabelung zwischen der Computertomographie-Gantry und dem Fahrwagen kann direkt erfolgen und muss insbesondere nicht durch das Drehlager geführt werden. Damit wird weniger Kabellänge benötig. Außerdem wirken damit auf die Kabel keine Wechselbelastungen aufgrund einer Drehung der Computertomographie-Gantry relativ zu dem Fahrwagen, sodass weniger anspruchsvolle Kabel verwendet werden. Weiterhin ist man im Design der Kabelverlegung freier, was einen Kosten- und Funktionsvorteil bietet. Auch ist die Zugänglichkeit für Montage und Service besser.

Insbesondere kann vorgesehen sein, dass die Computertomographie-Gantry relativ zu dem Fahrwagen in Bezug auf die vertikale Drehachse fest angeordnet ist, insbesondere gegen eine Winkelveränderung gesichert ist. Somit sind Maßnahmen zum Absichern von Scherbewegungen der Computertomographie-Gantry relativ zu dem Fahrwagen nicht erforderlich.

Dadurch, dass die Computertomographie-Gantry in den Fahrwagen direkt und nicht mittels des Drehlagers aufgenommen ist, während sich das System in dem Translations-Betriebszustand befindet, ist die mechanische Stabilität und damit auch die Bildqualität verbessert. Insbesondere können Schwingungen der Computertomographie-Gantry in dem Translations-Betriebszustand vermieden werden. Durch das Abstellen des Fahrwagens auf dem Schienensystem nach der Rotationsbewegung wird eine präzise und wiederholgenaue Anordnung des Fahrwagens relativ zu dem Schienensystem in Bezug auf die vertikale Drehachse, insbesondere in Bezug auf die 0°-Position und die 180° Position, ermöglicht.

Durch die modulweise Bauart ist es einfach möglich Varianten des Fahrwagens mit und ohne Dreh-Funktion zu definieren, die sich nur über wenige Bauteile differenzieren. Das erhöht die Mehrfachverwendung, senkt die Produkt- und Produktlebenszyklus-Kosten. Das Hub-Dreh-Modul kann beispielsweise einzeln gefertigt, geprüft und geliefert werden. Es ist möglich das Modul erst in dem Untersuchungsraum vor Ort zu installieren und/oder auszutauschen. Damit wird der Fahrwagen leichter und damit besser zu handhaben. Das Modul kann einzeln, insbesondere außerhalb des Fahrwagens, gewartet werden.

Im Rahmen der Erfindung können Merkmale, welche in Bezug auf unterschiedliche Ausführungsformen der Erfindung und/oder unterschiedliche Anspruchskategorien (Verfahren, Verwendung, Vorrichtung, System, Anordnung usw.) beschrieben sind, zu weiteren Ausführungsformen der Erfindung kombiniert werden. Beispielsweise kann ein Anspruch, der ein System betrifft, auch mit Merkmalen, die im Zusammenhang mit einem Verfahren beschrieben oder beansprucht sind, weitergebildet werden und umgekehrt. Funktionale Merkmale eines Verfahrens können dabei durch entsprechend ausgebildete gegenständliche Komponenten ausgeführt werden. Die Verwendung des unbestimmten Artikels "ein" bzw. "eine" schließt nicht aus, dass das betroffene Merkmal auch mehrfach vorhanden sein kann. Der Ausdruck "basierend auf" kann im Kontext der vorliegenden Anmeldung insbesondere im Sinne des Ausdrucks "unter Verwendung von" verstanden werden. Insbesondere schließt eine Formulierung, der zufolge ein erstes Merkmal basierend auf einem zweiten Merkmal berechnet (alternativ: ermittelt, generiert etc.) wird, nicht aus, dass das erste Merkmal ferner basierend auf einem dritten Merkmal berechnet (alternativ: ermittelt, generiert etc.) werden kann.

Im Folgenden wird die Erfindung anhand von Ausführungsbeispielen unter Hinweis auf die beigefügten Figuren erläutert. Die Darstellung in den Figuren ist schematisch, stark vereinfacht und nicht zwingend maßstabsgetreu.

Die Fig. 1 zeigt ein System mit einer Computertomographie-Gantry, einem Fahrwagen und einem Schienensystem in einem Translations-Betriebszustand des Systems.

Die Fig. 2 zeigt das System mit der Computertomographie-Gantry, dem Fahrwagen und dem Schienensystem in einem Rotations-Betriebszustand des Systems.

Die Fig. 3 zeigt einen beispielhaften Fahrwagen für das System.

Die Fig. 4 zeigt ein System ohne Hub-Dreh-Modul.

Die Fig. 5 zeigt ein Ablaufdiagramm eines Verfahrens zum Bewegen einer Computertomographie-Gantry.

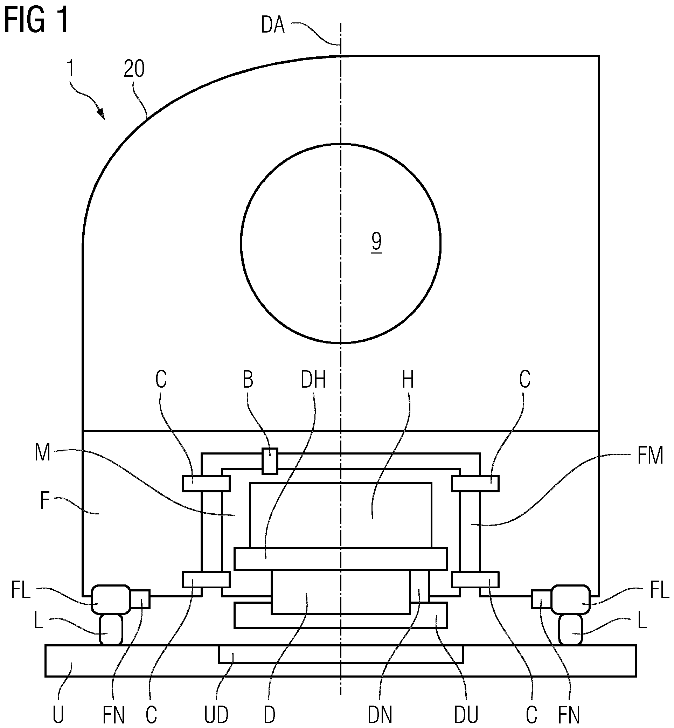

Die Fig. 1 zeigt das System 1, aufweisend die Computertomographie-Gantry 20, den Fahrwagen F, das Schienensystem L, das Drehlager D und die Hubvorrichtung H in einem Translations-Betriebszustand des Systems 1, wobei in dem Translations-Betriebszustand des Systems 1 der Fahrwagen F entlang des Schienensystems L derart bewegbar gelagert ist, dass entlang des Schienensystems L eine erste Translationsbewegung des Fahrwagens F ausführbar ist, wobei die Computertomographie-Gantry 20, das Drehlager D und die Hubvorrichtung H jeweils derart in den Fahrwagen F aufgenommen sind, dass sie der ersten Translationsbewegung des Fahrwagens F folgen. Die Computertomographie-Gantry 20 weist die Öffnung 9 auf.

In einem Übergangs-Betriebszustand des Systems 1 ist der Fahrwagen F mittels der Hubvorrichtung H relativ zu dem Schienensystem L entlang einer vertikalen Drehachse DA derart bewegbar gelagert, dass entlang der vertikalen Drehachse DA eine Hubbewegung des Fahrwagens F relativ zu dem Schienensystem L ausführbar ist, wobei die Computertomographie-Gantry 20 derart in den Fahrwagen F aufgenommen ist, dass sie der Hubbewegung des Fahrwagens F relativ zu dem Schienensystem L folgt. Das System 1 weist einen Fahrantrieb FN und einen Drehantrieb DN auf. Der Fahrantrieb FN ist zum Antreiben der ersten Translationsbewegung des Fahrwagens F und/oder zum Antreiben der zweiten Translationsbewegung des Fahrwagens F eingerichtet. Der Drehantrieb DN ist zum Antreiben der Rotationsbewegung des Fahrwagens F relativ zu dem Schienensystem L um die vertikale Drehachse DA eingerichtet.

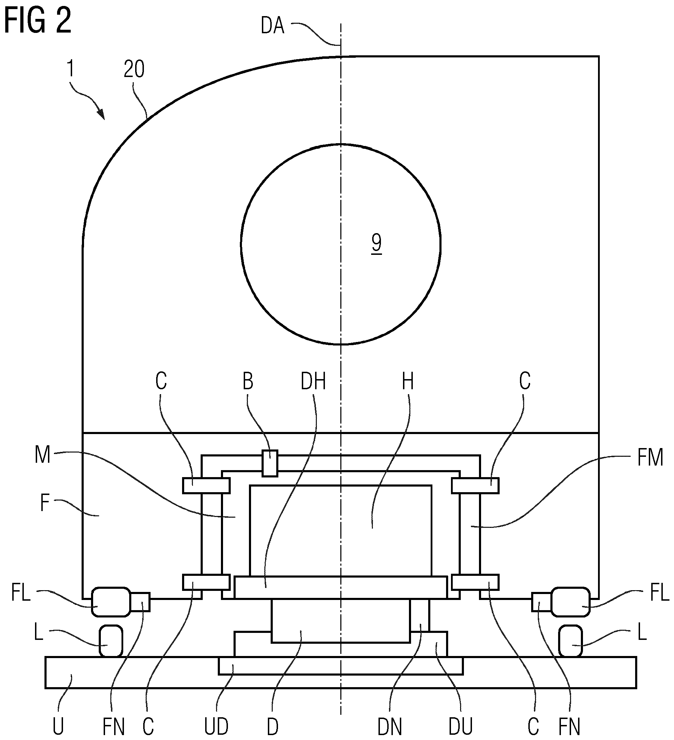

Die Fig. 2 zeigt das System 1 aufweisend die Computertomographie-Gantry 20, den Fahrwagen F, das Schienensystem L, das Drehlager D und die Hubvorrichtung H in einem Rotations-Betriebszustand des Systems 1, wobei in dem Rotations-Betriebszustand des Systems 1 der Fahrwagen F mittels der Hubvorrichtung H relativ zu dem Schienensystem L entlang der vertikalen Drehachse DA derart angehoben ist, dass der Fahrwagen F von dem Schienensystem L losgelöst ist, und der Fahrwagen F mittels des Drehlagers D relativ zu dem Schienensystem L um die vertikale Drehachse DA derart drehbar gelagert ist, dass eine Rotationsbewegung des Fahrwagens F relativ zu dem Schienensystem L um die vertikale Drehachse DA ausführbar ist, wobei die Computertomographie-Gantry 20 derart in den Fahrwagen F aufgenommen ist, dass sie der Rotationsbewegung des Fahrwagens F relativ zu dem Schienensystem L um die vertikale Drehachse DA folgt.

Das Schienensystem L weist einen Satz von Schienen auf, wobei der Fahrwagen F einen Satz von Rädern FL aufweist, wobei die

Rotationsbewegung des Fahrwagens F relativ zu dem Schienensystem L um die vertikale Drehachse DA von einem ersten Winkel um die vertikale Drehachse DA bis zu einem zweiten Winkel um die vertikale Drehachse DA erfolgt, wobei der Satz von Rädern FL in Bezug auf die vertikale Drehachse DA derart angeordnet ist, dass der Satz von Rädern FL auf dem Satz von Schienen rollen kann, wenn der Fahrwagen F relativ zu dem Schienensystem L in dem ersten Winkel um die vertikale Drehachse DA angeordnet ist, und dass der Satz von Rädern FL auf dem Satz von Schienen rollen kann, wenn der Fahrwagen F relativ zu dem Schienensystem L in dem zweiten Winkel um die vertikale Drehachse DA angeordnet ist.

Das System weist ferner die Stützstruktur UD auf, wobei das Schienensystem L relativ zu der Stützstruktur UD ruht, wobei in dem Translations-Betriebszustand des Systems 1 der Fahrwagen F entlang des Schienensystems L derart relativ zu der Stützstruktur UD bewegbar gelagert ist, dass entlang des Schienensystems L die erste Translationsbewegung des Fahrwagens F relativ zu der Stützstruktur UD ausführbar ist, wobei in dem Übergangs-Betriebszustand des Systems 1 und in dem Rotations-Betriebszustand des Systems 1 der Fahrwagen F mittels der Hubvorrichtung H und des Drehlagers D auf die Stützstruktur UD abgestützt ist.

Das System 1 weist die Sockelstruktur DU auf, wobei die Sockelstruktur DU mittels der Hubvorrichtung H relativ zu dem Fahrwagen F entlang der vertikalen Drehachse DA bewegbar gelagert ist, wobei die Sockelstruktur DU mittels des Drehlagers D relativ zu dem Fahrwagen F um die vertikale Drehachse DA drehbar gelagert ist, wobei die Hubvorrichtung H dazu eingerichtet ist, ausgehend von dem Translations-Betriebszustand des Systems 1 einen Abstand zwischen der Sockelstruktur DU und dem Fahrwagen F entlang der vertikalen Drehachse DA zu vergrößern und durch ein Stemmen der Sockelstruktur DU gegen die Stützstruktur UD den Fahrwagen F relativ zu dem Schienensystem L entlang der vertikalen Drehachse DA derart anzuheben, dass der Fahrwagen F von dem Schienensystem L losgelöst ist, wobei durch das Stemmen der Sockelstruktur DU gegen die Stützstruktur UD die Sockelstruktur DU derart gegen eine Winkeländerung um die vertikale Drehachse DA relativ zu der Stützstruktur UD fixiert ist, dass der Fahrwagen F mittels des Drehlagers D relativ zu der Stützstruktur UD um die vertikale Drehachse DA drehbar gelagert ist, sodass das System 1 in den Rotations-Betriebszustand des Systems 1 übergeht.

Durch das Stemmen der Sockelstruktur DU gegen die Stützstruktur UD ist die Sockelstruktur DU derart gegen die Winkeländerung um die vertikale Drehachse DA relativ zu der Stützstruktur UD reibschlüssig fixiert, dass der Fahrwagen F mittels des Drehlagers D relativ zu der Stützstruktur UD um die vertikale Drehachse DA drehbar gelagert ist.

Die Oberseite der Stützstruktur UD ist im Wesentlichen plan. Die Unterseite der Sockelstruktur DU ist im Wesentlichen plan. Das Stemmen der Sockelstruktur DU gegen die Stützstruktur UD bewirkt einen Reibschluss zwischen der Oberseite der Stützstruktur UD und der Unterseite der Sockelstruktur DU, wobei durch den Reibschluss die Sockelstruktur DU derart gegen die Winkeländerung um die vertikale Drehachse DA relativ zu der Stützstruktur UD reibschlüssig fixiert ist, dass der Fahrwagen F mittels des Drehlagers D relativ zu der Stützstruktur UD um die vertikale Drehachse DA drehbar gelagert ist.

Die Hubvorrichtung H ist dazu eingerichtet, ausgehend von dem Rotations-Betriebszustand des Systems 1 den Abstand zwischen der Sockelstruktur DU und dem Fahrwagen F entlang der vertikalen Drehachse DA zu verkleinern und dadurch den Fahrwagen F relativ zu dem Schienensystem L entlang der vertikalen Drehachse DA bis zu einem Abstellen des Fahrwagens F auf dem Schienensystem L abzusenken, sodass das System 1 in den Translations-Betriebszustand des Systems 1 übergeht.

Die Hubvorrichtung H und das Drehlager D sind direkt aneinander gekoppelt, sodass sie ein Hub-Dreh-Modul M bilden, wobei der Fahrwagen F eine Aussparung FM zur Aufnahme des Hub-Dreh-Moduls M aufweist, wobei das Hub-Dreh-Modul M in der Aussparung derart angeordnet ist, dass sich die Hubvorrichtung H entlang der vertikalen Drehachse DA nach unten aus der Aussparung FM herausstrecken kann, um den Fahrwagen F relativ zu dem Schienensystem L entlang der vertikalen Drehachse DA anzuheben.

Das Hub-Dreh-Modul M ist über eine mechanische Schnittstelle C und eine elektrische Schnittstelle B mit dem Fahrwagen F lösbar verbunden. Dadurch kann das Hub-Dreh-Modul M mit geringem Aufwand von dem Fahrwagen F getrennt bzw. darin montiert werden. Das Hub-Dreh-Modul M kann insbesondere in Form eines Stempels ausgebildet sein.

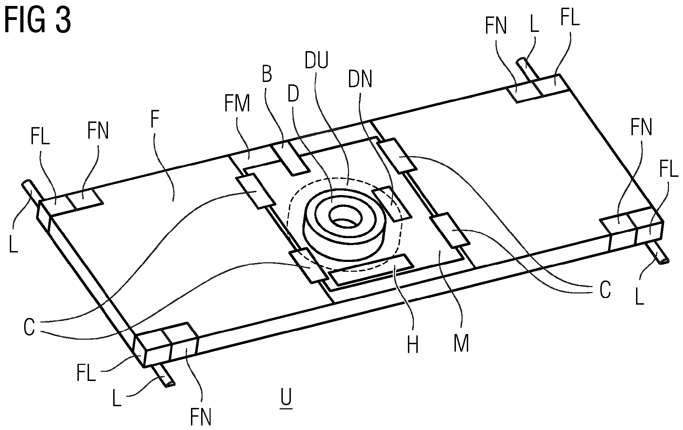

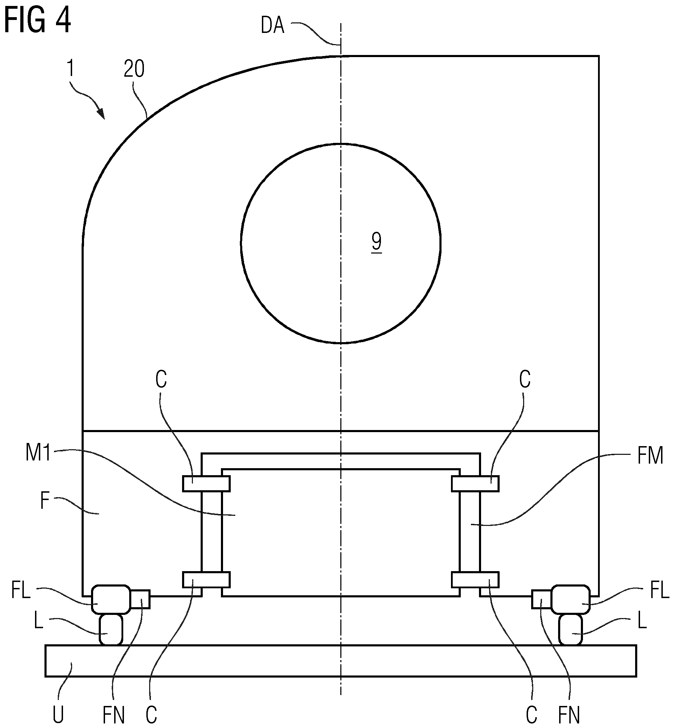

Die Fig. 3 zeigt einen beispielhaften Fahrwagen F für das System 1. Die Fig. 4 zeigt ein System ohne das Hub-Dreh-Modul M. Statt des Hub-Dreh-Moduls M ist darin lediglich das Strukturelement M1 verbaut.

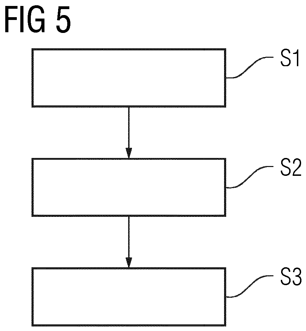

Die Fig. 5 zeigt ein Ablaufdiagramm eines Verfahrens zum Bewegen der Computertomographie-Gantry 20, das Verfahren umfassend:
- ein Ausführen S1 einer ersten Translationsbewegung eines Fahrwagens F entlang eines Schienensystems L, während sich ein System 1, welches die Computertomographie-Gantry 20, den Fahrwagen F, das Schienensystem L, ein Drehlager D und eine Hubvorrichtung H aufweist, in einem Translations-Betriebszustand des Systems 1 befindet, wobei in dem Translations-Betriebszustand des Systems 1 der Fahrwagen F entlang des Schienensystems L bewegbar gelagert ist, wobei die Computertomographie-Gantry 20, das Drehlager D und die Hubvorrichtung H jeweils derart in den Fahrwagen F aufgenommen sind, dass sie der ersten Translationsbewegung des Fahrwagens F folgen,
- ein Ausführen S2 einer Hubbewegung des Fahrwagens F relativ zu dem Schienensystem L entlang einer vertikalen Drehachse DA, während sich das System 1 in einem Übergangs-Betriebszustand des Systems 1 befindet, wobei in dem Übergangs-Betriebszustand des Systems 1 der Fahrwagen F mittels der Hubvorrichtung H relativ zu dem Schienensystem L entlang einer vertikalen Drehachse DA bewegbar gelagert ist, wobei die Computertomographie-Gantry 20 derart in den Fahrwagen F aufgenommen ist, dass sie der Hubbewegung des Fahrwagens F relativ zu dem Schienensystem L folgt,
- ein Ausführen S3 einer Rotationsbewegung des Fahrwagens F relativ zu dem Schienensystem L um die vertikale Drehachse DA, während sich das System 1 in einem Rotations-Betriebszustand des Systems 1 befindet, wobei in dem Rotations-Betriebszustand des Systems 1 der Fahrwagen F mittels der Hubvorrichtung H relativ zu dem Schienensystem L entlang der vertikalen Drehachse DA derart angehoben ist, dass der Fahrwagen F von dem Schienensystem L losgelöst ist, und der Fahrwagen F mittels des Drehlagers D relativ zu dem Schienensystem L um die vertikale Drehachse DA drehbar gelagert ist, wobei die Computertomographie-Gantry 20 derart in den Fahrwagen F aufgenommen ist, dass sie der Rotationsbewegung des Fahrwagens F relativ zu dem Schienensystem L um die vertikale Drehachse DA folgt.

## Patentansprüche

1. System (1), aufweisend einen Fahrwagen (F), in den eine Computertomographie-Gantry (20) aufnehmbar ist, ein Schienensystem (L), ein Drehlager (D) und eine Hubvorrichtung (H),
- wobei in einem Translations-Betriebszustand des Systems (1) der Fahrwagen (F) entlang des Schienensystems (L) derart bewegbar gelagert ist, dass entlang des Schienensystems (L) eine erste Translationsbewegung des Fahrwagens (F) ausführbar ist, wobei das Drehlager (D) und die Hubvorrichtung (H) jeweils derart in den Fahrwagen (F) aufgenommen sind, dass sie der ersten Translationsbewegung des Fahrwagens (F) folgen,
- wobei in einem Übergangs-Betriebszustand des Systems (1) der Fahrwagen (F) mittels der Hubvorrichtung (H) relativ zu dem Schienensystem (L) entlang einer vertikalen Drehachse (DA) derart bewegbar gelagert ist, dass entlang der vertikalen Drehachse (DA) eine Hubbewegung des Fahrwagens (F) relativ zu dem Schienensystem (L) ausführbar ist,
- wobei in einem Rotations-Betriebszustand des Systems (1) der Fahrwagen (F) mittels der Hubvorrichtung (H) relativ zu dem Schienensystem (L) entlang der vertikalen Drehachse (DA) derart angehoben ist, dass der Fahrwagen (F) von dem Schienensystem (L) losgelöst ist, und der Fahrwagen (F) mittels des Drehlagers (D) relativ zu dem Schienensystem (L) um die vertikale Drehachse (DA) derart drehbar gelagert ist, dass eine Rotationsbewegung des Fahrwagens (F) relativ zu dem Schienensystem (L) um die vertikale Drehachse (DA) ausführbar ist,
- wobei die Computertomographie-Gantry (20) der ersten Translationsbewegung des Fahrwagens (F), der Hubbewegung des Fahrwagens (F) relativ zu dem Schienensystem (L) und der Rotationsbewegung des Fahrwagens (F) relativ zu dem Schienensystem (L) um die vertikale Drehachse (DA) folgt, wenn die Computertomographie-Gantry (20) in den Fahrwagen (F) aufgenommen ist.

2. System (1) nach Anspruch 1,
- wobei das Schienensystem (L) einen Satz von Schienen aufweist,
- wobei der Fahrwagen (F) einen Satz von Rädern (FL) aufweist,
- wobei die Rotationsbewegung des Fahrwagens (F) relativ zu dem Schienensystem (L) um die vertikale Drehachse (DA) von einem ersten Winkel um die vertikale Drehachse (DA) bis zu einem zweiten Winkel um die vertikale Drehachse (DA) erfolgt,
- wobei der Satz von Rädern (FL) in Bezug auf die vertikale Drehachse (DA) derart angeordnet ist, dass der Satz von Rädern (FL) auf dem Satz von Schienen rollen kann, wenn der Fahrwagen (F) relativ zu dem Schienensystem (L) in dem ersten Winkel um die vertikale Drehachse (DA) angeordnet ist, und dass der Satz von Rädern (FL) auf dem Satz von Schienen rollen kann, wenn der Fahrwagen (F) relativ zu dem Schienensystem (L) in dem zweiten Winkel um die vertikale Drehachse (DA) angeordnet ist.

3. System (1) nach Anspruch 2,
- wobei ein Absolutbetrag einer Winkeldifferenz zwischen dem ersten Winkel um die vertikale Drehachse (DA) und dem zweiten Winkel um die vertikale Drehachse (DA) größer als 0° und kleiner als 360° ist.

4. System (1) nach einem der Ansprüche 1 bis 3, ferner aufweisend eine Stützstruktur (UD),
- wobei das Schienensystem (L) relativ zu der Stützstruktur (UD) ruht,
- wobei in dem Translations-Betriebszustand des Systems (1) der Fahrwagen (F) entlang des Schienensystems (L) derart relativ zu der Stützstruktur (UD) bewegbar gelagert ist, dass entlang des Schienensystems (L) die erste Translationsbewegung des Fahrwagens (F) relativ zu der Stützstruktur (UD) ausführbar ist,
- wobei in dem Übergangs-Betriebszustand des Systems (1) und in dem Rotations-Betriebszustand des Systems (1) der Fahrwagen (F) mittels der Hubvorrichtung (H) und des Drehlagers (D) auf die Stützstruktur (UD) abgestützt ist.

5. System (1) nach Anspruch 4,
- wobei das System (1) eine Sockelstruktur (DU) aufweist,
- wobei die Sockelstruktur (DU) mittels der Hubvorrichtung (H) relativ zu dem Fahrwagen (F) entlang der vertikalen Drehachse (DA) bewegbar gelagert ist,
- wobei die Sockelstruktur (DU) mittels des Drehlagers (D) relativ zu dem Fahrwagen (F) um die vertikale Drehachse (DA) drehbar gelagert ist,
- wobei die Hubvorrichtung (H) dazu eingerichtet ist, ausgehend von dem Translations-Betriebszustand des Systems (1) einen Abstand zwischen der Sockelstruktur (DU) und dem Fahrwagen (F) entlang der vertikalen Drehachse (DA) zu vergrößern und durch ein Stemmen der Sockelstruktur (DU) gegen die Stützstruktur (UD) den Fahrwagen (F) relativ zu dem Schienensystem (L) entlang der vertikalen Drehachse (DA) derart anzuheben, dass der Fahrwagen (F) von dem Schienensystem (L) losgelöst ist, wobei durch das Stemmen der Sockelstruktur (DU) gegen die Stützstruktur (UD) die Sockelstruktur (DU) derart gegen eine Winkeländerung um die vertikale Drehachse (DA) relativ zu der Stützstruktur (UD) fixiert ist, dass der Fahrwagen (F) mittels des Drehlagers (D) relativ zu der Stützstruktur (UD) um die vertikale Drehachse (DA) drehbar gelagert ist, sodass das System (1) in den Rotations-Betriebszustand des Systems (1) übergeht.

6. System (1) nach Anspruch 5,
- wobei durch das Stemmen der Sockelstruktur (DU) gegen die Stützstruktur (UD) die Sockelstruktur (DU) derart gegen die Winkeländerung um die vertikale Drehachse (DA) relativ zu der Stützstruktur (UD) reibschlüssig fixiert ist, dass der Fahrwagen (F) mittels des Drehlagers (D) relativ zu der Stützstruktur (UD) um die vertikale Drehachse (DA) drehbar gelagert ist.

7. System (1) nach Anspruch 6,
- wobei eine Oberseite der Stützstruktur (UD) im Wesentlichen plan ist,
- wobei eine Unterseite der Sockelstruktur (DU) im Wesentlichen plan ist,
- wobei das Stemmen der Sockelstruktur (DU) gegen die Stützstruktur (UD) einen Reibschluss zwischen der Oberseite der Stützstruktur (UD) und der Unterseite der Sockelstruktur (DU) bewirkt,
- wobei durch den Reibschluss die Sockelstruktur (DU) derart gegen die Winkeländerung um die vertikale Drehachse (DA) relativ zu der Stützstruktur (UD) reibschlüssig fixiert ist, dass der Fahrwagen (F) mittels des Drehlagers (D) relativ zu der Stützstruktur (UD) um die vertikale Drehachse (DA) drehbar gelagert ist.

8. System (1) nach einem der Ansprüche 5 bis 7,
- wobei die Hubvorrichtung (H) dazu eingerichtet ist, ausgehend von dem Rotations-Betriebszustand des Systems (1) den Abstand zwischen der Sockelstruktur (DU) und dem Fahrwagen (F) entlang der vertikalen Drehachse (DA) zu verkleinern und dadurch den Fahrwagen (F) relativ zu dem Schienensystem (L) entlang der vertikalen Drehachse (DA) bis zu einem Abstellen des Fahrwagens (F) auf dem Schienensystem (L) abzusenken, sodass das System (1) in den Translations-Betriebszustand des Systems (1) übergeht.

9. System (1) nach einem der Ansprüche 1 bis 8,
- wobei die Hubvorrichtung (H) und das Drehlager (D) direkt aneinandergekoppelt sind, sodass sie ein Hub-Dreh-Modul (M) bilden,
- wobei der Fahrwagen (F) eine Aussparung (FM) zur Aufnahme des Hub-Dreh-Moduls (M) aufweist,
- wobei das Hub-Dreh-Modul (M) in der Aussparung derart angeordnet ist, dass sich die Hubvorrichtung (H) entlang der vertikalen Drehachse (DA) nach unten aus der Aussparung (FM) herausstrecken kann, um den Fahrwagen (F) relativ zu dem Schienensystem (L) entlang der vertikalen Drehachse (DA) anzuheben.

10. Verfahren zum Bewegen eines Fahrwagens (F), das Verfahren umfassend:
- ein Ausführen (S1) einer ersten Translationsbewegung eines Fahrwagens (F), in den eine Computertomographie-Gantry (20) aufnehmbar ist, entlang eines Schienensystems (L), während sich ein System (1), welches den Fahrwagen (F), das Schienensystem (L), ein Drehlager (D) und eine Hubvorrichtung (H) aufweist, in einem Translations-Betriebszustand des Systems (1) befindet, wobei in dem Translations-Betriebszustand des Systems (1) der Fahrwagen (F) entlang des Schienensystems (L) bewegbar gelagert ist, wobei das Drehlager (D) und die Hubvorrichtung (H) jeweils derart in den Fahrwagen (F) aufgenommen sind, dass sie der ersten Translationsbewegung des Fahrwagens (F) folgen,
- ein Ausführen (S2) einer Hubbewegung des Fahrwagens (F) relativ zu dem Schienensystem (L) entlang einer vertikalen Drehachse (DA), während sich das System (1) in einem Übergangs-Betriebszustand des Systems (1) befindet, wobei in dem Übergangs-Betriebszustand des Systems (1) der Fahrwagen (F) mittels der Hubvorrichtung (H) relativ zu dem Schienensystem (L) entlang einer vertikalen Drehachse (DA) bewegbar gelagert ist,
- ein Ausführen (S3) einer Rotationsbewegung des Fahrwagens (F) relativ zu dem Schienensystem (L) um die vertikale Drehachse (DA), während sich das System (1) in einem Rotations-Betriebszustand des Systems (1) befindet, wobei in dem Rotations-Betriebszustand des Systems (1) der Fahrwagen (F) mittels der Hubvorrichtung (H) relativ zu dem Schienensystem (L) entlang der vertikalen Drehachse (DA) derart angehoben ist, dass der Fahrwagen (F) von dem Schienensystem (L) losgelöst ist, und der Fahrwagen (F) mittels des Drehlagers (D) relativ zu dem Schienensystem (L) um die vertikale Drehachse (DA) drehbar gelagert ist,
- wobei die Computertomographie-Gantry (20) der ersten Translationsbewegung des Fahrwagens (F), der Hubbewegung des Fahrwagens (F) relativ zu dem Schienensystem (L) und der Rotationsbewegung des Fahrwagens (F) relativ zu dem Schienensystem (L) um die vertikale Drehachse (DA) folgt, wenn die Computertomographie-Gantry (20) in den Fahrwagen (F) aufgenommen ist.

11. Verfahren nach Anspruch 10,
- wobei nach dem Ausführen (S3) der Rotationsbewegung des Fahrwagens (F) relativ zu dem Schienensystem (L) um die vertikale Drehachse (DA) eine Senkbewegung des Fahrwagens (F) relativ zu dem Schienensystem (L) entlang der vertikalen Drehachse (DA) ausgeführt wird, während sich das System (1) in dem Übergangs-Betriebszustand des Systems (1) befindet,
- wobei die Computertomographie-Gantry (20) der Senkbewegung des Fahrwagens (F) relativ zu dem Schienensystem (L) folgt, wenn die Computertomographie-Gantry (20) in den Fahrwagen (F) aufgenommen ist.

12. Verfahren nach Anspruch 11,
- wobei nach einem Ausführen der Senkbewegung des Fahrwagens (F) relativ zu dem Schienensystem (L) entlang der vertikalen Drehachse (DA) eine zweite Translationsbewegung des Fahrwagens (F) entlang des Schienensystems (L) ausgeführt wird, während sich das System (1) in dem Translations-Betriebszustand des Systems (1) befindet, wobei das Drehlager (D) und die Hubvorrichtung (H) jeweils derart in den Fahrwagen (F) aufgenommen sind, dass sie der zweiten Translationsbewegung des Fahrwagens (F) folgen,
- wobei das Schienensystem (L) einen Satz von Schienen aufweist,
- wobei der Fahrwagen (F) einen Satz von Rädern (FL) aufweist,
- wobei die Rotationsbewegung des Fahrwagens (F) relativ zu dem Schienensystem (L) um die vertikale Drehachse (DA) von einem ersten Winkel um die vertikale Drehachse (DA) bis zu einem zweiten Winkel um die vertikale Drehachse (DA) erfolgt,
- wobei während der ersten Translationsbewegung des Fahrwagens (F) entlang des Schienensystems (L) der Fahrwagen (F) relativ zu dem Schienensystem (L) in dem ersten Winkel um die vertikale Drehachse (DA) angeordnet ist und der Satz von Rädern (FL) auf dem Satz von Schienen rollt,
- wobei während der zweiten Translationsbewegung des Fahrwagens (F) entlang des Schienensystems (L) der Fahrwagen (F) relativ zu dem Schienensystem (L) in dem zweiten Winkel um die vertikale Drehachse (DA) angeordnet ist und der Satz von Rädern (FL) auf dem Satz von Schienen rollt.

13. Verfahren nach einem der Ansprüche 10 bis 12,
- wobei das System (1) ferner eine Stützstruktur (UD) aufweist, wobei das Schienensystem (L) relativ zu der Stützstruktur (UD) ruht,
- wobei die erste Translationsbewegung des Fahrwagens (F) entlang des Schienensystems (L) relativ zu der Stützstruktur (UD) ausgeführt wird, während sich das System (1) in dem Translations-Betriebszustand des Systems (1) befindet,
- wobei nach dem Ausführen (S1) der ersten Translationsbewegung des Fahrwagens (F) entlang des Schienensystems (L) relativ zu der Stützstruktur (UD) der Fahrwagen (F) mittels der Hubvorrichtung (H) und des Drehlagers (D) auf die Stützstruktur (UD) abgestützt wird.

14. Verfahren nach Anspruch 13,
- wobei das System (1) eine Sockelstruktur (DU) aufweist, wobei die Sockelstruktur (DU) mittels der Hubvorrichtung (H) relativ zu dem Fahrwagen (F) entlang der vertikalen Drehachse (DA) bewegbar gelagert ist, wobei die Sockelstruktur (DU) mittels des Drehlagers (D) relativ zu dem Fahrwagen (F) um die vertikale Drehachse (DA) drehbar gelagert ist,
- wobei ausgehend von dem Translations-Betriebszustand des Systems (1) mittels der Hubvorrichtung (H) ein Abstand zwischen der Sockelstruktur (DU) und dem Fahrwagen (F) entlang der vertikalen Drehachse (DA) vergrößert wird und durch ein Stemmen der Sockelstruktur (DU) gegen die Stützstruktur (UD) der Fahrwagen (F) relativ zu dem Schienensystem (L) entlang der vertikalen Drehachse (DA) derart angehoben wird, dass der Fahrwagen (F) von dem Schienensystem (L) losgelöst wird, wobei durch das Stemmen der Sockelstruktur (DU) gegen die Stützstruktur (UD) die Sockelstruktur (DU) gegen eine Drehung um die vertikale Drehachse (DA) relativ zu der Stützstruktur (UD) fixiert wird.

15. Verfahren nach Anspruch 14,
- wobei durch das Stemmen der Sockelstruktur (DU) gegen die Stützstruktur (UD) die Sockelstruktur (DU) gegen eine Drehung um die vertikale Drehachse (DA) relativ zu der Stützstruktur (UD) reibschlüssig fixiert wird.
